# EUROPEAN PATENT APPLICATION

(11) **EP 3 979 254 A1**
(43) Date of publication of application: **06.04.2022**
(21) Application number: 20813122.7
(22) Date of filing: 18.05.2020
(51) Int. Cl.: G16H 15/00, G16H 50/00

(54) **MEDICAL INFORMATION REGISTRATION SUPPORT DEVICE, MEDICAL INFORMATION REGISTRATION SUPPORT METHOD, AND PROGRAM**

(30) Priority: 30.05.2019 JP 2019101351
(71) Applicant: FUJIFILM Toyama Chemical Co., Ltd., Chuo-ku Tokyo 104-0031 (JP)
(72) Inventor: HANEDA, Shinji, Tokyo 104-0031 (JP)
(74) Representative: Dewhurst, Joseph Michael
(86) International application number: PCT/JP2020/019573
(87) International publication number: WO 2020/241332

(57) **Abstract**

The present invention has an object to provide a medical information registration support device, a medical information registration support method, and a program that allow a user to easily register medical information. A medical information registration support device according to a first aspect of the present invention includes an input accepting unit configured to accept input of medical information, a determining unit configured to determine whether the accepted medical information and registration information match each other, a candidate generating unit configured to generate a candidate for the registration information for the accepted medical information by using a trained model that is constructed through machine learning, standardizes input medical information according to a type of the medical information and outputs the standardized medical information when the accepted medical information and the registration information do not match each other, and a registering unit for registering, as the registration information, medical information selected from the candidate or medical information obtained by correcting the candidate in association with the accepted medical information.

## Description

### BACKGROUND OF THE INVENTION

### 1. Field of the Invention

The present invention relates to a medical information registration support device, a medical information registration support method, and a program that that support registration of medical information.

### 2. Description of the Related Art

In a medical field, medical information is used in various devices and systems. In that case, medical practice to be performed by medical doctors and the like, results of the medical practice, and medical materials and pharmaceutical products to be used are registered and used in various devices and systems of medical institutions. For example, in the case of medicines (pharmaceutical products) prescribed to patients, information on usage (usage code, usage name) corresponding to an individual medicine is used in a medical institution system (for example, an electronic medical record, a receipt, an audit support system). For example, Patent Literature 1 describes that a medical care support device displays a list of usage codes according to each of categories of medicines, and a user selects and edits a usage code to input the usage.

### Citation List

Patent Literature 1: Japanese Patent Application Laid-Open No. 2009-020772

### SUMMARY OF THE INVENTION

With respect to medicines, there are many usage names because there are a large number of medicines that can be prescribed to patients, and there are variations in usage name to be input by medical institutions such as hospitals and pharmacies and users (doctors, pharmacists) even if the actual usage is the same. In addition, the medicine and the usage name do not always have a one-to-one correspondence relation, and the usage name may differ even for the same medicine depending on a medical condition or a doctor's or pharmacist's view. In such a situation, if all are registered as input, a large number of similar usage names may be registered, or information may be insufficient or duplicated, which may cause confusion. Furthermore, according to the purpose of use (audit support, etc.), it may be necessary to convert and/or decompose a usage to and/or into a dosing timing(s) to register the usage. As described above, the registration of usage names is a work that imposes heavy loads on users, but prior arts such as Patent Literature 1 does not take such a situation into consideration. The problem of "there are variations in expression, etc. of information to be input, and thus the load on registration is high" also exists for medical information other than the usage names of medicines.

The present invention has been made in view of such circumstances, and has an object to provide a medical information registration support device, a medical information registration support method, and a program that enable a user to easily register medical information.

In order to attain the above object, a medical information registration support device according to a first aspect of the present invention comprises: an input accepting unit configured to accept input of medical information; a determining unit configured to determine whether the accepted medical information and registration information match each other; a candidate generating unit configured to generate a candidate for the registration information for the accepted medical information by using a trained model that is constructed through machine learning, standardizes input medical information according to a type of the medical information and outputs the standardized medical information when the accepted medical information and the registration information do not match each other; and a registering unit configured to register, as the registration information, medical information selected from the candidate or medical information obtained by correcting the candidate in association with the accepted medical information.

In the first aspect, when the medical information accepted by the input accepting unit and the registered medical information do not match each other (including a case where the accepted medical information has not been registered), the candidate generating unit generates a candidate for the registration information for the accepted medical information by using the trained model that is constructed through machine learning, standardizes the input medical information according to the type of the medical information and outputs the standardized medical information. Therefore, by selecting or correcting the candidate, a user can easily register the standardized medical information even when there are variations in the expression, etc. of the input medical information. Note that the "standardization" of medical information includes outputting medical information according to a predetermined standard. Further, the candidate generating unit can use a trained model constructed by using, for example, a deep learning algorithm as a machine learning method.

According to a medical information registration support device of a second aspect, in the first aspect, the input accepting unit accepts, as the medical information, information which includes at least one of information indicating any of medical practice and/or a result of the medical practice, a medical material and a pharmaceutical product as a registration target, and information indicating how the registration target is executed, acquired, or used. The second aspect defines specific aspects and configurations of the medical information. As "the information indicating any of medical practice and/or a result of the medical practice, a medical material and a pharmaceutical product as a registration target" (an example of information indicating the classification of the medical information), examples of the "medical practice" may include the names and operative methods of surgery, medical examination, consultation, diagnosis, technique, and treatment, and examples of the "result of medical practice" may include a diagnosis name, a finding, etc. Further, examples of the "medical material" may include the types and names of medical materials to be used in surgery, medical examination, etc. and medical materials to be used by patients. Examples of "pharmaceutical product" may include the types and names of pharmaceutical products to be used in surgery, medical examination, etc., and pharmaceutical products to be taken by patients.

Further, examples of "information indicating how the registration target is executed, acquired, or used" may include the conditions, number of times, timings, etc. of "execution, acquisition, use", and they may differ according to the classification of the registration target (which one of the medical practice, the result of medical practice, the medical material, and the pharmaceutical product).

According to a medical information registration support device of a third aspect, in the first or second aspect, the input accepting unit accepts input of information indicating a usage name of a medicine as the medical information, and the candidate generating unit generates a candidate for a dosing timing of the medicine as a candidate for the registration information. The usage name of a medicine (one aspect of the medical information) includes information such as the dosing condition, the dosing frequency, the dosing timing, etc., but there may be variations in the expression of the usage name among users such as doctors and pharmacists, or medical institutions (pharmacies, etc.) that prescribe the medicine. Therefore, the load of registration is high in the prior art such as the above-mentioned Patent Literature 1. However, according to the third aspect, the user can easily register the usage names of medicines.

According to a medical information registration support device of a fourth aspect, in the third aspect, the determining unit makes the determination by referring to a conversion table in which information indicating usage names of medicines and dosing timings of the medicines are recorded in association with each other. The fourth aspect defines one aspect of a method for determining whether the medical information and the registration information match each other. Information indicating usage names of medicines and usage types may be registered in association with each other in the conversion table.

According to a medical information registration support device of a fifth aspect, in the third or fourth aspect, the trained model includes a first trained model configured to estimate a usage type of the medicine based on the accepted usage name, and a second trained model configured to estimate a dosing timing of the medicine based on the usage type. The fifth aspect defines one aspect of the configuration of the trained model. The second trained model may use the usage type estimated by the first trained model, or may use a separately estimated or input usage type.

According to a medical information registration support device of a sixth aspect, in the fifth aspect, the candidate generating unit generates a candidate for the usage type by associating the usage type with the accepted usage name and the estimated dosing timing, and the registering unit registers a usage type selected from the generated candidate for the usage type or a usage type obtained by correcting the candidate for the usage type in association with the accepted usage name. According to the sixth aspect, the usage name, the usage type, and the dosing timing are registered in association with one another. When generating a usage type candidate, the candidate generating unit may use a usage type estimated by the first trained model or may use a separately estimated or input usage type.

According to a medical information registration support device of a seventh aspect, in the fifth or sixth aspect, the candidate generating unit generates time-point information indicating a dosing time point for the medicine from the accepted usage name according to the usage type, and sets the generated time-point information as a dosing timing candidate. In the seventh aspect, the candidate generating unit can generate time-point information in which one time point indicates one dosing.

According to a medical information registration support device of an eighth aspect, in any one of the fifth to seventh aspects, the first trained model and the second trained model are trained models constructed based on an algorithm of natural language processing. The algorithm of natural language processing is effective in processing information expressed in letters or numbers like usage names.

According to a medical information registration support device of a ninth aspect, in any one of the fifth to eighth aspects, the first trained model and the second trained model are trained models constructed by a neural network. In the ninth aspect, for example, a recurrent neural network (RNN) can be used as the neural network.

A medical information registration support device of a tenth aspect further comprises a learning unit configured to cause the trained model to perform additional learning based on the correction in any one of the first to ninth aspects, and the candidate generating unit generates the candidate by using the trained model in which the additional learning has been performed. According to the tenth aspect, the accuracy of the generation of the registration information candidate can be improved by the additional learning.

A medical information registration support device according to an eleventh aspect further comprises a data generating unit configured to generate additional learning data based on the correction in the tenth aspect, and the learning unit causes to perform additional learning using the additional learning data. The learning unit can cause to perform the learning by using the corrected data as correct answer data (teacher data).

A medical information registration support device according to a twelfth aspect further comprises a normalizing unit configured to normalize the accepted medical information in any one of the first to eleventh aspects, and the determining unit makes the determination based on the normalized medical information. Examples of normalization may include the conversion between fullwidth and halfwidth forms, the conversion between Chinese numerals and Arabic numerals, the conversion of different fonts of kanji characters to a unified word, etc. in the expression of the medical information, and it is possible to improve the accuracy of the determination and generation of candidates by these processing.

In order to attain the above object, a medical information registration support method according to a thirteenth aspect of the present invention comprises: an input accepting step of accepting input of medical information; a determining step of determining whether the accepted medical information and registration information match each other; a candidate generating step of generating a candidate for the registration information for the accepted medical information by using a trained model that is constructed through machine learning, standardizes input medical information according to a type of the medical information and outputs the standardized medical information when the accepted medical information and the registration information do not match each other; and a registering step of registering, as the registration information, medical information selected from the candidate or medical information obtained by correcting the candidate in association with the accepted medical information. According to the thirteenth aspect, the user can easily register the medical information as in the first aspect. Further, the medical information registration support method according to the thirteenth aspect may further have a configuration similar to each of those of the second to twelfth aspects.

In order to attain the above object, a program according to a fourteenth aspect of the present invention causes a computer to execute the medical information registration support method according to the thirteenth aspect. According to the fourteenth aspect, the user can easily register the medical information as in the first and thirteenth aspects. Further, in the fourteenth aspect, the program may cause the computer to execute a medical information registration support method further having a configuration similar to each of those of the second to twelfth aspects. A non-transient recording medium in which computer-readable codes of these programs are recorded can also be mentioned as an aspect of the present invention.

As described above, according to the medical information registration support device, the medical information registration support method, and the program of the present invention, a user can easily register medical information.

### BRIEF DESCRIPTION OF THE DRAWINGS

Figure 1 is a block diagram showing a configuration of a usage name registration support device according to a first embodiment.
Figure 2 is a functional block diagram of a processing unit.
Figure 3 is a flowchart showing a procedure of usage name registration processing.
Figure 4 is a diagram showing examples of a usage code and a usage name.
Figure 5 is a diagram showing an example of a conversion table.
Figure 6 is a diagram showing the structure of an RNN
Figure 7 is a diagram showing examples of a time point estimated by a trained model.
Figure 8 is a diagram showing examples of a candidate estimated by the trained model.
Figure 9 is a diagram showing examples of a candidate and registration information.
Figure 10 is a diagram showing an example of additional learning data.
Figure 11 is a diagram showing a state of one-dose-packaging audit support using a registered usage name.
Figure 12 is a diagram showing an example of audited packaged bags with labels.

### DESCRIPTION OF THE PREFERRED EMBODIMENTS

Embodiments of a usage name registration support device, a usage name registration support method, and a program according to the present invention will be described in detail as follows. The description will be made with reference to the accompanying drawings.

### <First Embodiment

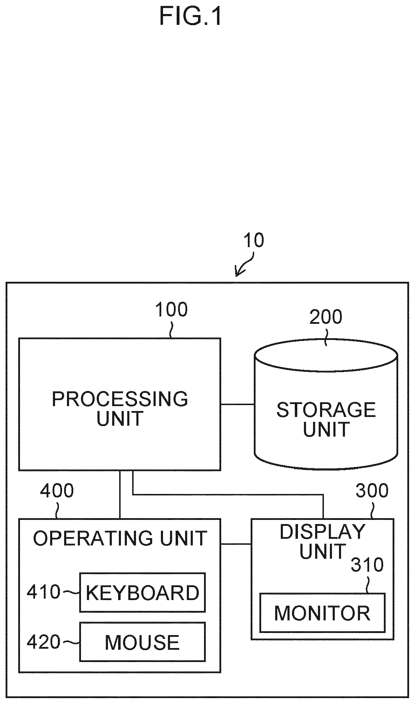

Figure 1 is a block diagram showing a configuration of a usage name registration support device 10 (medical information registration support device) according to a first embodiment. The usage name registration support device 10 is a device for supporting registration of a usage name (one aspect of medical information) of a medicine, and can be implemented by using a computer. As shown in Figure 1, the usage name registration support device 10 includes a processing unit 100, a storage unit 200, a display unit 300, and an operating unit 400, and these units are connected to one another to transmit and receive necessary information among these units. Various installation modes can be adopted for these components, and each component may be installed at one place (in one housing, one room or the like), or may be installed at a distant place and connected via a network. Further, the usage name registration support device 10 may be connected to an in-hospital system (not shown), an external server, an external database, or the like via a network (not shown) to acquire information as needed.

### <Configuration of processing unit>

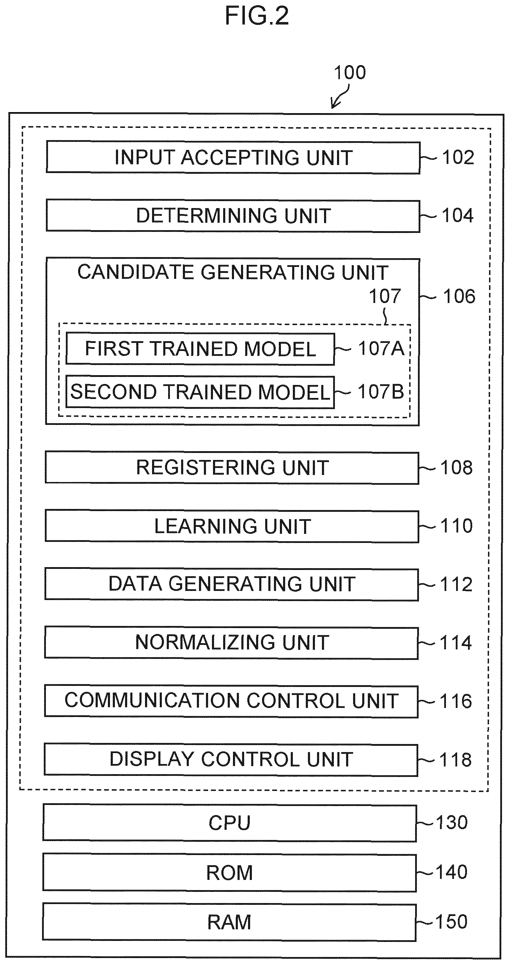

Figure 2 is a diagram showing a configuration of the processing unit 100. The processing unit 100 includes an input accepting unit 102 (input accepting unit), a determining unit 104 (determining unit), a candidate generating unit 106 (candidate generating unit), a registering unit 108 (registering unit), a learning unit 110 (learning unit), a data generating unit 112 (data generating unit), a normalizing unit 114 (normalizing unit), a communication control unit 116, and a display control unit 118. The processing unit 100 further includes a CPU 130 (CPU: Central Processing Unit), a ROM 140 (ROM: Read Only Memory), and a RAM 150 (RAM: Random Access Memory). Note that the processing by each of these units is performed under the control of the CPU 130.

The functions of the respective units of the processing unit 100 described above can be implemented by using various processors. The various processors include, for example, a CPU which is a general-purpose processor for executing software (program) to implement various functions. In addition, the various processors described above also include a programmable logic device (PLD) which is a processor whose circuit configuration can be changed after manufacturing, such as a graphics processing unit (GPU) or a field programmable gate array (FPGA), which are specialized processors for image processing. Further, the above-mentioned various processors also include such as a dedicated electric circuit which is a processor having a circuit configuration specially designed for executing specific processing, such as an application specific integrated circuit (ASIC).

The function of each unit may be implemented by one processor, or may be implemented by a plurality of processors of the same type or different types (for example, a plurality of FPGAs, or a combination of a CPU and an FPGA, or a combination of a CPU and a GPU). Moreover, one processor may implement a plurality of functions. As examples in which a plurality of functions are configured by one processor include, there is a first mode in which one processor is configured by a combination of one or more CPUs and software and this processor implements a plurality of functions as represented by computers such as a client and a server, and also there is a second mode in which a processor for implementing the functions of the entire system with one IC (Integrated Circuit) chip is used as represented by system on chip (SoC). As described above, various functions are configured as hardware structures by using one or more of the above-mentioned various processors. Further, the hardware structures of these various processors are, more specifically, electric circuits (circuitry) in which circuit elements such as semiconductor elements are combined.

When the above-mentioned processors or electric circuits execute software (programs), codes of the software to be executed, which are readable by a computer (for example, various processors and electric circuits constituting the processing unit 100, and/or a combination thereof), have been stored in a non-transient recording medium such as ROM 140 (see Figure 2), and the processors refers to the software. The software to be stored in the non-transient recording medium includes a program (usage name registration support program, medical information registration support program) for executing the usage name registration support method (medical information registration support method) according to the present invention. The code of the program may be recorded in non-transient recording media such as various optical magnetic recording devices and semiconductor memories instead of the ROM 140. In the case of processing using software, for example, the RAM 150 is used as a temporary storage area, and for example, data stored in an electronically erasable and programmable read only memory (EEPROM) (not shown) can be referred to.

### <Configuration of storage unit>

The storage unit 200 includes a non-transient recording medium such as a digital versatile disk (DVD), a hard disk, various semiconductor memories, and a control unit thereof, and registration information on usage names, additional learning data, and the like are stored in the storage unit 200.

### Configurations of display unit and operating unit>

The display unit 300 includes a monitor 310 (display device), and can display an input usage name (medical information), information stored in the storage unit 200, a processing result obtained by the processing unit 100, and the like. The operating unit 400 includes a keyboard 410 and a mouse 420 as input devices and/or pointing devices, and a user can perform an operation necessary for executing the usage name registration support method (medical information registration support method) according to the present invention via these devices and the screen of the monitor 310 (described later). The monitor 310 may be configured by a touch panel so that the user can perform the operation via the touch panel. Operations which the user can perform by using the operating unit 400 include, for example, correction of a candidate for registration information.

### <Processing of usage name registration support method>

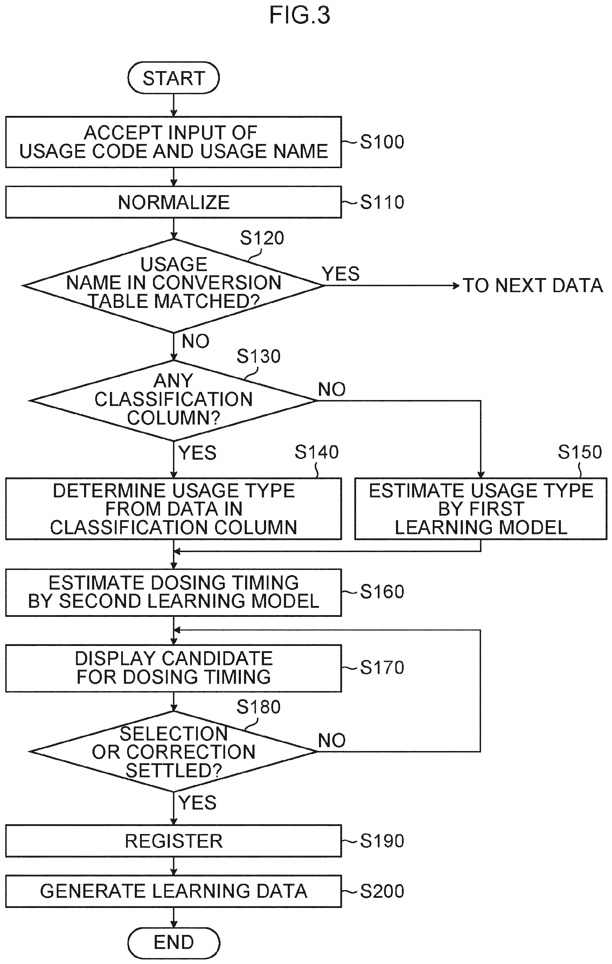

Hereinafter, the processing of the usage name registration support method by the usage name registration support device 10 having the above-described configuration will be described with reference to the flowchart of Figure 3.

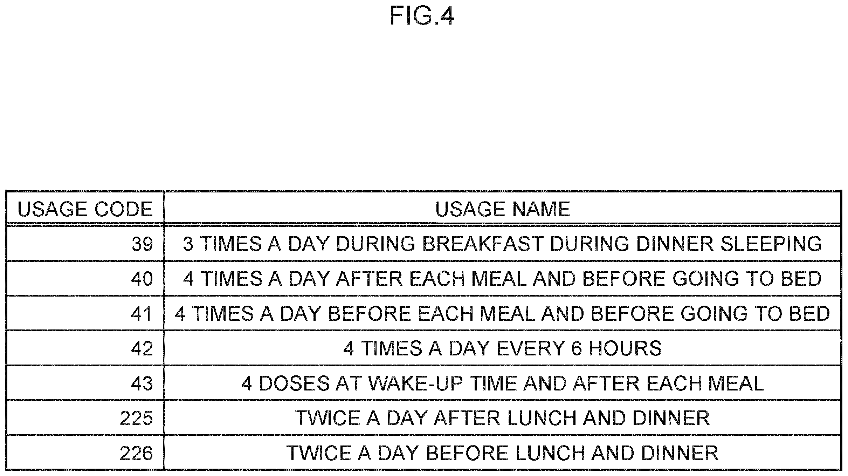

The input accepting unit 102 accepts input of a usage code and a usage name of a medicine (step S100: input accepting step). The usage name includes information indicating "how the medicine (pharmaceutical product) is used or taken", and it is one aspect of "medical information" in the present invention (with respect to medical information other than the usage name, see an item of "Registration support for other than usage name"). The usage name is determined by prescription data (information on a prescription read out by a prescription reader or the like, or information input and/or edited by a user such as a doctor or pharmacist based on the prescription). Figure 4 is a diagram showing examples of the usage code and the usage name. Further, other data (for example, data of "classification" described later) may be included in the input.

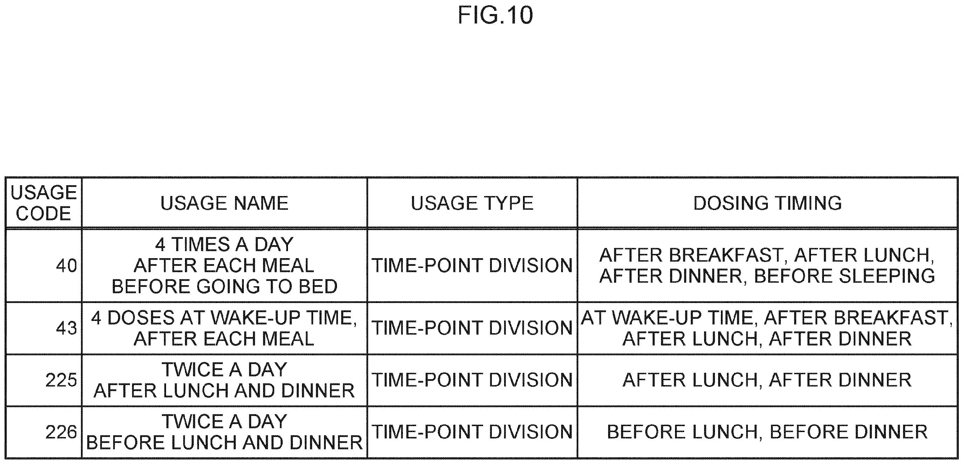

Note that the usage code and usage name to be input may be input by a user's operation via the operating unit 400, or may be obtained from another device or system (for example, a one-dose-packaging device; see Figure 10) via the communication control unit 116. Further, the usage name registration support device 10 may accept input of a plurality of data (usage codes and usage names) in a lump and perform processing in step S120 and subsequent steps for each data, or may input next data and perform the processing on the next data after inputting one data and completing the processing on the data.

The normalizing unit 114 normalizes medical information received in step S100 (step S110: normalizing step). Examples of "normalization" include the conversion between fullwidth and halfwidth forms, the conversion between Chinese numerals and Arabic numerals, etc. in the expression of the medical information, and the normalizing unit 114 performs these processing, whereby it is possible to improve the accuracy of the determination and generation of candidates (described later).

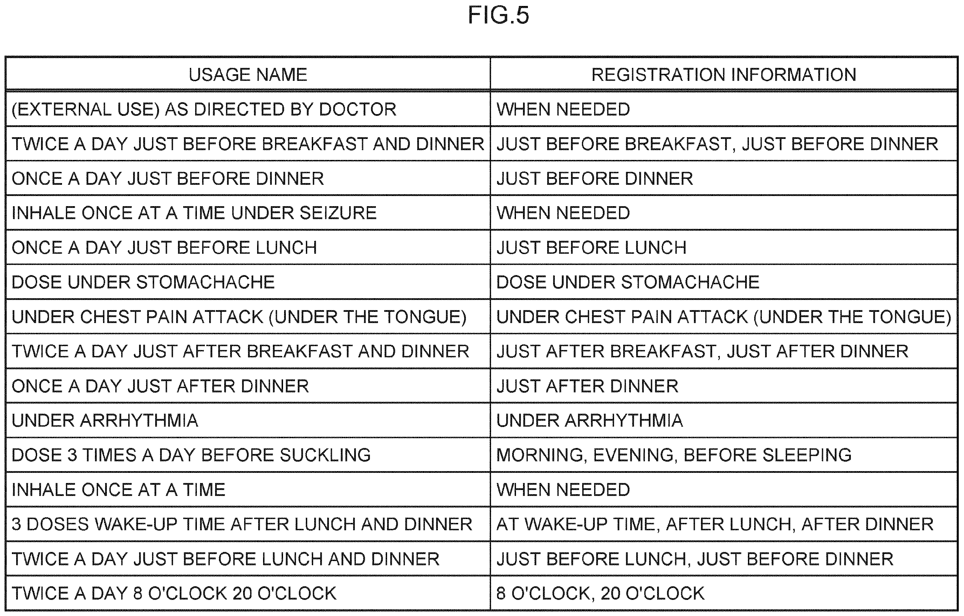

The determining unit 104 determines whether the usage name (medical information) and the registration information match each other (step S120: determining step). When the determination is made, the determining unit 104 refers to a conversion table in which information indicating the usage name of each medicine and the dosing timing of the medicine are recorded in association with each other. Figure 5 is a diagram showing an example of the conversion table. In the conversion table of Figure 5, the column of "Usage name" indicates input and normalized usage names, and the column of "Registration information" is information which has been standardized (in this example, the usage name is divided into individual dosing timings (time-points), and the dosing timings are separated by "commas"), and then registered. Here, Figure 5 shows an example in which the conversion table includes the usage names and the time-point-divided registration information thereof, but the conversion table may include information on usage types.

The determining unit 104 can affirm the determination in step S120 when the accepted medical information and the information in the conversion table completely match each other. When the determination is affirmed (YES in step S120), that is, the accepted medical information has been already registered, the processing of step S120 and subsequent steps are performed on next data (usage code and usage name).

### <Generation of candidate for registration information using trained model>

When the determination in step S120 is negated (when the accepted usage name (medical information) and the registration information do not match each other), the candidate generating unit 106 (candidate generating unit) generates candidates for registration information for the accepted usage name by using a trained model 107 constructed through machine learning (steps S130 to S170: candidate generating step). As described in detail below, the trained model 107 is a trained model for outputting candidates for the dosing timing from the input usage name (an example of standardization corresponding to the type of medical information). The trained model 107 includes a first trained model 107A (first trained model) for estimating the usage type of a medicine based on a usage name, and a second trained model 107B (second trained model) for estimating the dosing timing of the medicine based on the usage type (see Figure 2).

### <Configuration of trained model>

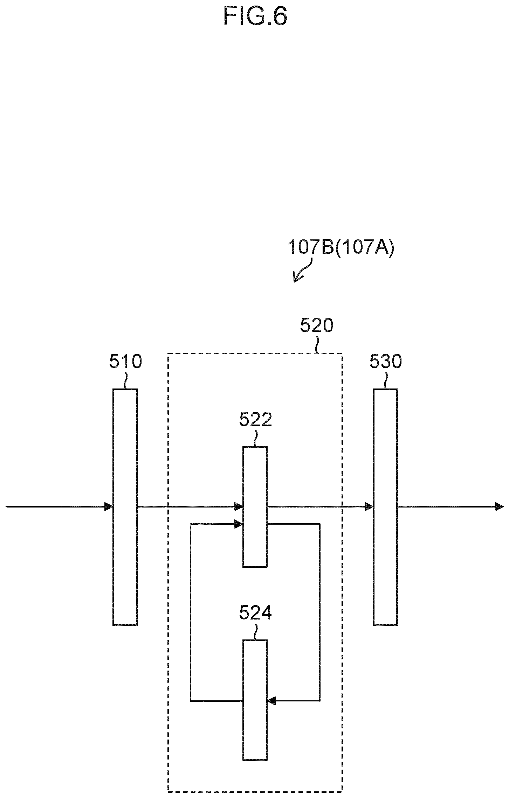

The trained model 107 (first trained model 107A and second trained model 107B) is a trained model which is constructed based on an algorithm of natural language processing. Specifically, the trained model 107 can be configured by a recurrent neural network (RNN one aspect of neural network). Figure 6 is a diagram showing a configuration example of the second trained model 107B configured by the RNN (a similar configuration can be adopted for the first trained model 107A). The second trained model 107B has an input layer 510, a hidden layer 520 and an output layer 530, and the hidden layer 520 is different from a normal neural network in that the hidden layer 520 has a hidden layer 522 indicating a state at a current time (time t) and a hidden layer 524 indicating a state at a past time (time t-1). The second trained model 107B holds the state of the hidden layer at time t-1 and uses it to input the next time t, so that it is possible to perform an estimation using past records of information input time-sequentially like natural language (in this embodiment, the context of characters and words in an accepted usage name). Note that the second trained model 107B may be configured by using a long short-term memory (LSTM) which is a kind of RNN

### <Machine learning using past registration content and the like as learning data>

For example, the learning unit 110 causes a neural network such as the RNN to perform machine learning using a past registration content as learning data, thereby making it possible to construct the trained model 107 (first trained model 107A and second trained model 107B). For this machine learning, the learning unit 110 can use, for example, learning data in which an input usage name (medical information) and data (registration information) registered based on the usage name are paired. Further, the learning unit 110 may use learning data in which a standard usage name and data corresponding to the usage name are paired (the same applies to additional learning described later).

### <Estimation of usage type>

The candidate generating unit 106 determines whether the input data has a classification column (step S130: candidate generating step). The classification column contains information indicating the usage type of a medicine, for example, "time-point division" type (take a medicine after breakfast, take a medicine before sleeping, etc.), "when needed" type (take a medicine at the time when abdominal pain occurs, take a medicine at the time when a fit of chest pain occurs, etc.), and "usage-name follow (following an input usage name as it is)" type ("4 times a day, every 6 hours", etc.). When the input data has a classification column (YES in step S130), the candidate generating unit 106 determines the usage type from data in the classification column (step S140: candidate generating step), and proceeds to step S160. On the other hand, when the input data has no classification column (NO in step S130), the candidate generating unit 106 estimates the usage type by using the first trained model 107A (step S150: candidate generating step). The first trained model 107A is a trained model for standardizing and outputting the input usage name (one aspect of medical information). Note that the "standardization" of medical information includes outputting medical information according to a predetermined standard, and the first trained model 107A can output any of predetermined usage types (for example, the above-mentioned "time-point division", "when needed" and "usage-name follow").

### <Estimation of dosing timing>

The candidate generating unit 106 estimates the dosing timing by using the second trained model 107B that estimates the dosing timing of a medicine based on the usage type (step S160: candidate generating step). In the estimation of the dosing timing, the candidate generating unit 106 inputs the usage type determined from the data in the classification column or the usage type estimated by using the first trained model 107A into the second trained model 107B. Further, the candidate generating unit 106 inputs the above-mentioned usage name (after normalization) into the second trained model 107B. The second trained model 107B is a trained model that standardizes and outputs the usage name (one aspect of medical information) according to the usage type. Here, the "standardization" of medical information includes outputting medical information according to a predetermined standard, and the second trained model 107B can perform the following processing.

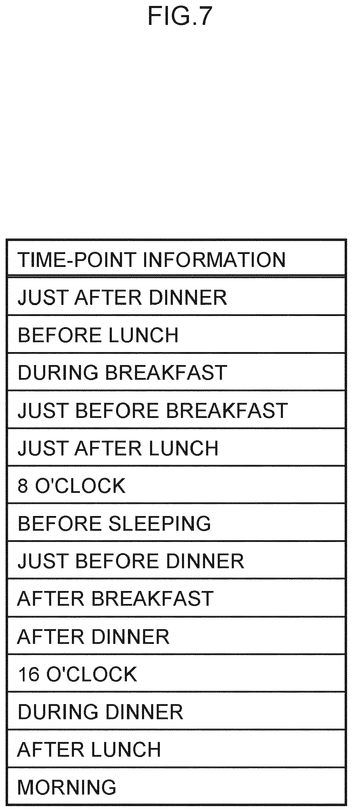

Figure 7 is a table showing an example of time-point information. Further, when the usage name contains a plurality of time-point information (when the medicine is taken a plurality of times a day), the second trained model 107B can, for example, separate respective time-point information by commas "," to list the time-point information, and output the listed time-point information. The generation of individual time-point information and the listing of the respective time-point information with commas are one aspect of the standardization of usage names (medical information). Further, a meal can be described like "morning meal", "meal in the morning", and "breakfast", for example. However, it is also one aspect of the standardization that when the second trained model 107B generates time-point information based on the timing of a meal, these expressions can be unified into any expression out of these expressions. In the example of Figure 7, the second trained model 107B unifies the expressions into "breakfast". Similarly, in the example of Figure 7, "before going to bed", "before bedtime", "before sleeping" and the like are unified into "before sleeping". In addition to the above-mentioned example, the second trained model 107B may unify the time in a 24-hour system.

The candidate generating unit 106 generates time-point information indicating the time point of dosing the medicine by the trained model 107 in this way, and uses the generated time-point information as a candidate for a dosing timing.

### <Selection or correction of candidate>

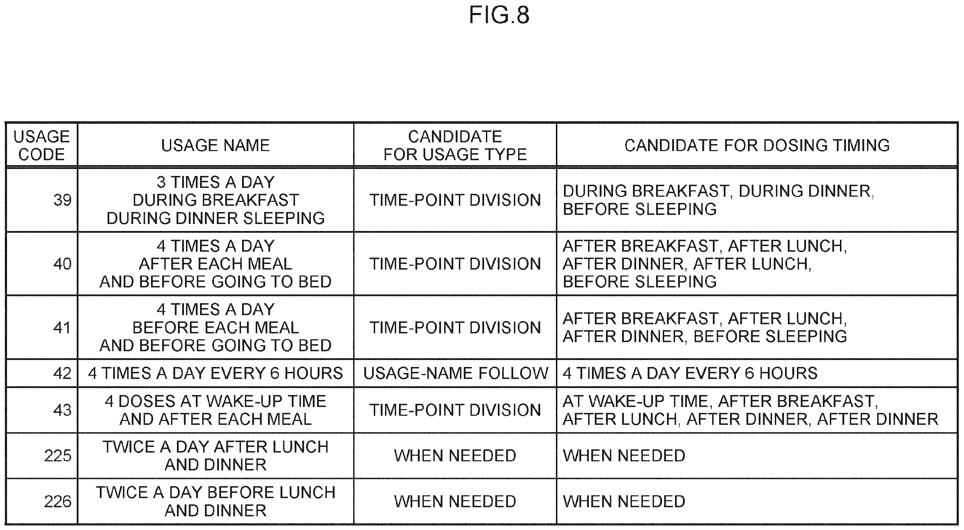

The candidate generating unit 106 and the display control unit 118 cause the monitor 310 (display device) to display the generated candidate for the dosing timing (step S170: candidate generating step, registration step). The candidate generating unit 106 and the display control unit 118 may display a candidate for the determined usage type or the estimated usage type together, or may display a plurality of candidates when there are the plurality of candidates. Figure 8 is a diagram showing examples of usage type candidates and dosing timing candidates (time-point information included in the usage names). The registering unit 108 accepts selection or correction of a candidate by a user's operation via the operating unit 400, which allows the user to correct an inappropriate candidate.

### <Registration of dosing timing>

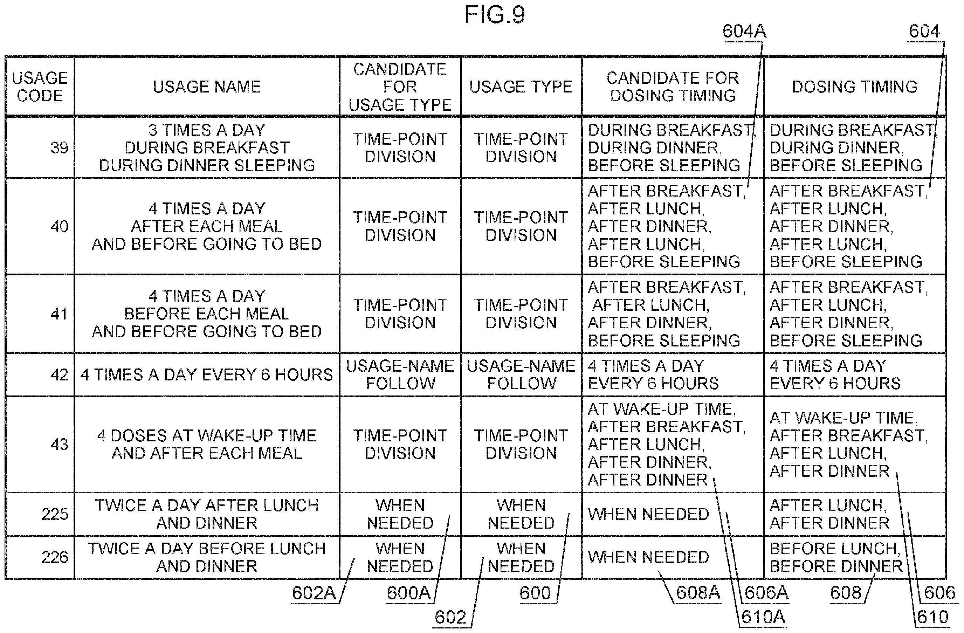

When the above-mentioned selection or correction is settled (YES in step S180: registration step), the settled dosing timing (registration information) and the usage name are registered in association with each other (step S190: registration step). The registering unit 108 may register a usage type selected from displayed usage type candidates or register a usage type obtained by correcting a usage type candidate in association with the accepted usage name (step S190: registration step). The registration in step S190 can be performed by the registering unit 108 updating the above-mentioned conversion table (adding data). Figure 9 is a diagram showing examples of candidates and settled information with respect to the usage type and the dosing timing. Note that in the example of Figure 9, the columns designated by reference numerals 600 to 610 indicate that candidates (columns designated by reference numerals 600A to 610A) have been corrected. For example, in the column designated by reference numeral 600A, the usage type is estimated to be "when needed", but it is corrected to be "time-point division" as indicated in the column designated by reference numeral 600. Further, in the column designated by reference numeral 610A, the dosing timing is estimated to be "at wake-up time, after breakfast, after lunch, after dinner, after dinner", but it is corrected to be "at wake-up time, after breakfast, after lunch, after dinner" as indicated in the column designated by reference numeral 610.

As described above, in the first embodiment, a dosing timing (registration information) candidate is estimated from an input usage name, and the usage name and the dosing timing are registered in association with each other. Therefore, the user can easily register usage names (medical information) without requiring the user to register the usage names individually.

### <Generation of additional learning data and additional learning>

When the selection or correction for the above-mentioned dosing timing candidate is settled, the data generating unit 112 (data generating unit) generates additional learning data based on the settled selection or correction (step S200: data generating step). The data generating unit 112 can determine "selection or correction has been settled" according to a user's operation (for example, clicking an enter button) via the operating unit 400. The data generating unit 112 may also generate additional learning data for the usage type.

The data generating unit 112 can create additional learning data for usage names whose candidates have been corrected because they have been incorrectly estimated. For example, in the example of Figure 9, since the candidates are corrected in the columns designated by reference numerals 600 to 610, additional learning data (correct answer data, teacher data) are created for these data (data whose usage codes are 40, 43, 225, and 226). Figure 10 is an example of additional learning data for the usage type and the dosing timing. The learning unit 110 (learning unit) causes to perform additional learning using additional learning data (additional learning step). The learning unit 110 may cause to perform additional learning every time additional learning data is generated, or may cause the trained model 107 to perform additional learning periodically or at any time according to a user's instruction. Such additional learning makes it possible to improve the estimation accuracy by the trained model 107. Here, the additional learning step is not shown in the flowchart of Figure 3.

### <Utility form of usage name registration support device, etc.>

### <One-dose-packaging audit support using registered usage name>

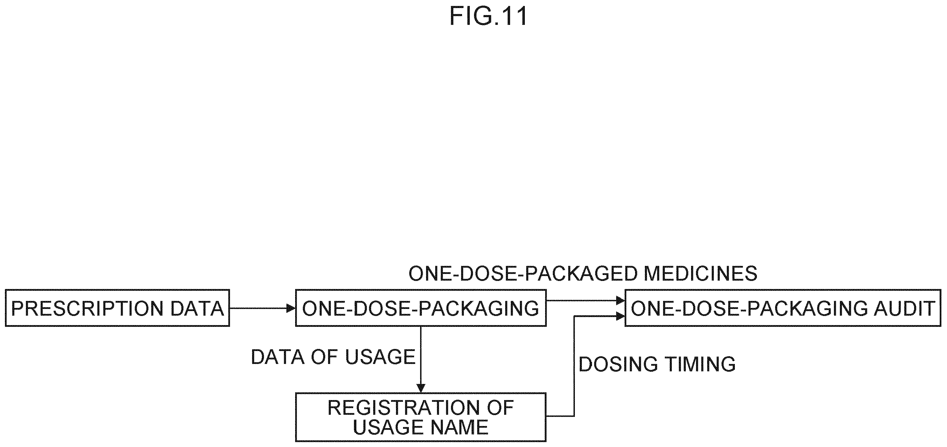

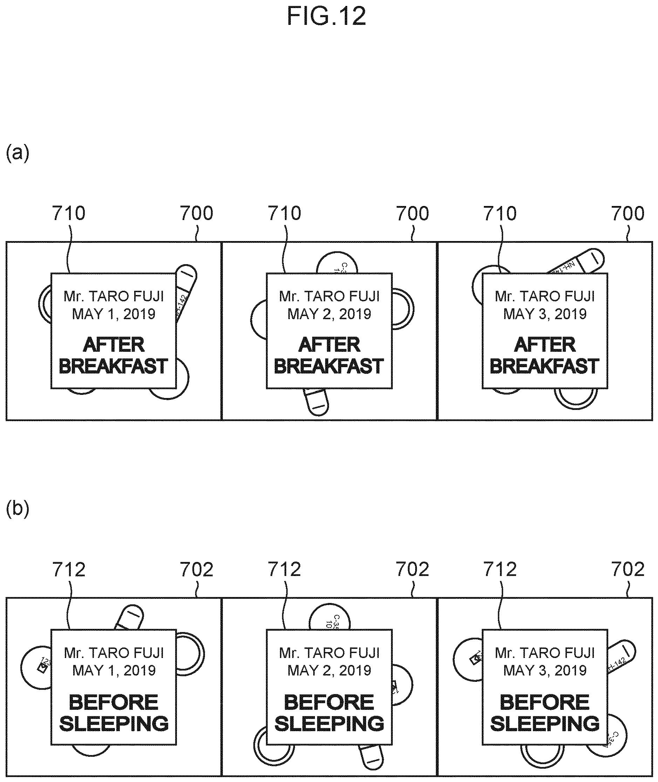

The usage name registration support device, the usage name registration support method, and the program according to the above-described embodiment can be used, for example, in the following modes. Figure 11 is a diagram showing the flow of one-dose-packaging audit support. A doctor prepares a prescription, and the doctor or a pharmacist corrects the prescription as necessary to create prescription data. Based on this prescription data, medicines to be taken at one time are packaged in one dose (packaged separately). One-dose-packaging is performed by a one-dose-packaging device, and the usage is digitalized (usage codes, usage names, etc.) by the one-dose-packaging device. This data is registered by the usage name registration support device 10 or the like to obtain data on the dosing timing. The audit support device determines whether the one-dose-packaged medicines match the prescription data, and prints or labels dosing timings on packaged bags for which "match the prescription data" is determined. Figure 12 is a diagram showing a state in which labels are affixed to the audited packaged bags. Part (a) of Figure 12 shows a state in which labels 710 printed with "after breakfast" are affixed to packaged bags 700 of medicines to be taken after breakfast, and part (b) of Figure 12 shows a state in which labels 712 printed with "before sleeping" are affixed to packaged bags 702 of medicines to be taken before sleeping. The dosing timings indicated on the labels 710 and 712 are the dosing timings registered by the usage name registration support device 10 (in this example, time-point-divided).

### <Registration support other than usage name>

The medical information registration support device, the medical information registration support method, and the program of the present invention can process not only the usage names of medicines described in the first embodiment, but also various medical information. Here, "medical information" is information which includes at least one of information indicating any of medical practice and/or a result of the medical practice, a medical material and a pharmaceutical product as a registration target, and information indicating how the registration target is executed, acquired, or used. With respect to the registration targets, examples of "medical practice" may include the names and operative methods of surgery, medical examination, consultation, diagnosis, technique, and treatment, and examples of "result of medical practice" may include results of surgery and medical examination, diagnosis names, disease names, symptom names, findings, etc. Further, examples of the "medical material" may include the types and names of medical materials to be used in surgery, medical examination, etc. and medical materials to be used by patients. With respect to "pharmaceutical product", it may include tablet or capsule type solid medicines, liquid medicines such as injections, and gaseous medicines, and may include the types and names of not only medicines to be taken (internally) by patients, but also medicines which are used by external use such as eye drops, application, and attachment. Further, "pharmaceutical product" may include the types and names of pharmaceutical products to be used in surgery or medical examination. In addition, "information indicating how the registration targets are executed, acquired, or used" may include the condition, frequency, timing, etc. of "executed, acquired, used", and they may be varied according to the type of registration target (any of the medical practice, the result of the medical practice, the medical material, or the pharmaceutical product).

According to the medical information registration support device, the medical information registration support method, and the program of the present invention, the user can easily register medical information even when there are variations in expression and the like of the medical information.

Although the embodiments and other examples of the present invention have been described above, the present invention is not limited to the above-described aspects, and various modifications can be made without departing from the spirit of the present invention.

### Explanation of References

- 10: usage name registration support device
- 100: processing unit
- 102: input accepting unit
- 104: determining unit
- 106: candidate generating unit
- 107: trained model
- 107A: first trained model
- 107B: second trained model
- 108: registering unit
- 110: learning unit
- 112: data generating unit
- 114: normalizing unit
- 116: communication control unit
- 118: display control unit
- 130: CPU
- 140: ROM
- 150: RAM
- 200: storage unit
- 300: display unit
- 310: monitor
- 400: operating unit
- 410: keyboard
- 420: mouse
- 510: input layer
- 520: hidden layer
- 522: hidden layer
- 524: hidden layer
- 530: output layer
- 700: packaged bag
- 702: packaged bag
- 710: label
- 712: label
- S100 to S200: steps of usage name registration support method (medical information registration support method)

## Claims

1. A medical information registration support device comprising:
an input accepting unit configured to accept input of medical information;
a determining unit configured to determine whether the accepted medical information and registration information match each other;
a candidate generating unit configured to generate a candidate for the registration information for the accepted medical information by using a trained model that is constructed through machine learning, standardizes input medical information according to a type of the medical information and outputs the standardized medical information when the accepted medical information and the registration information do not match each other; and
a registering unit configured to register, as the registration information, medical information selected from the candidate or medical information obtained by correcting the candidate in association with the accepted medical information.

2. The medical information registration support device according to claim 1, wherein the input accepting unit accepts, as the medical information, information which includes at least one of information indicating any of medical practice and/or a result of the medical practice, a medical material and a pharmaceutical product as a registration target, and information indicating how the registration target is executed, acquired, or used.

3. The medical information registration support device according to claim 1 or 2, wherein the input accepting unit accepts input of information indicating a usage name of a medicine as the medical information, and the candidate generating unit generates a dosing timing candidate for the medicine as a candidate for the registration information.

4. The medical information registration support device according to claim 3, wherein the determining unit makes the determination by referring to a conversion table in which information indicating usage names of medicines and dosing timings of the medicines are recorded in association with each other.

5. The medical information registration support device according to claim 3 or 4, wherein the trained model includes a first trained model configured to estimate a usage type of the medicine based on the accepted usage name, and a second trained model configured to estimate a dosing timing of the medicine based on the usage type.

6. The medical information registration support device according to claim 5, wherein the candidate generating unit generates a candidate for the usage type by associating the usage type with the accepted usage name and the estimated dosing timing, and the registering unit registers a usage type selected from the generated candidate for the usage type or a usage type obtained by correcting the candidate for the usage type in association with the accepted usage name.

7. The medical information registration support device according to claim 5 or 6, wherein the candidate generating unit generates time-point information indicating a dosing time point for the medicine from the accepted usage name according to the usage type, and sets the generated time-point information as a candidate for the dosing timing.

8. The medical information registration support device according to any one of claims 5 to 7, wherein the first trained model and the second trained model are trained models constructed based on an algorithm of natural language processing.

9. The medical information registration support device according to any one of claims 5 to 8, wherein the first trained model and the second trained model are trained models constructed by a neural network.

10. The medical information registration support device according to any one of claims 1 to 9, further comprising a learning unit configured to cause the trained model to perform additional learning based on the correction, wherein the candidate generating unit generates the candidate by using the trained model in which the additional learning has been performed.

11. The medical information registration support device according to claim 10, further comprising a data generating unit configured to generate additional learning data based on the correction, wherein the learning unit causes the trained model to perform additional learning using the additional learning data.

12. The medical information registration support device according to any one of claims 1 to 11, further comprising a normalizing unit configured to normalize the accepted medical information, wherein the determining unit makes the determination based on the normalized medical information.

13. A medical information registration support method comprising:
an input accepting step of accepting input of medical information;
a determining step of determining whether the accepted medical information and registration information match each other;
a candidate generating step of generating a candidate for the registration information for the accepted medical information by using a trained model that is constructed through machine learning, standardizes input medical information according to a type of the medical information and outputs the standardized medical information when the accepted medical information and the registration information do not match each other; and
a registering step of registering, as the registration information, medical information selected from the candidate or medical information obtained by correcting the candidate in association with the accepted medical information.

14. A program causing a computer to execute the medical information registration support method according to claim 13.

15. A non-transient and computer-readable recording medium that causes a computer to execute the program according to claim 14 when a command stored in the recording medium is read by the computer.
